# EUROPÄISCHE PATENTSCHRIFT

(11) **EP 3 796 025 B1**
(45) Veröffentlichungstag und Bekanntmachung des Hinweises auf die Patenterteilung: **18.06.2025**
(21) Anmeldenummer: 19198324.6
(22) Anmeldetag: 19.09.2019
(51) Int. Cl.: G01R 33/54, G01R 33/28, A61B 5/055

(54) **MAGNETRESONANZVORRICHTUNG MIT EINEM BEWEGBAREN PATIENTENTISCH UND EINER PATIENTENTISCHSTEUERUNG SOWIE EIN VERFAHREN ZU EINEM STEUERN EINER BEWEGUNG EINES PATIENTENTISCHS**
MAGNETIC RESONANCE DEVICE COMPRISING A MOVABLE PATIENT TABLE AND A PATIENT TABLE CONTROL, AND A METHOD FOR CONTROLLING A MOVEMENT OF A PATIENT TABLE
DISPOSITIF DE RÉSONANCE MAGNÉTIQUE DOTÉ D'UNE TABLE POUR LE PATIENT MOBILE D'UNE COMMANDE DE TABLE POUR LE PATIENT AINSI QUE PROCÉDÉ DE COMMANDE D'UN MOUVEMENT D'UNE TABLE POUR LE PATIENT

(43) Veröffentlichungstag der Anmeldung: 24.03.2021
(73) Patentinhaber: Siemens Healthineers AG, 91301 Forchheim (DE)
(72) Erfinder: Biber, Stephan, 91056 Erlangen (DE)
(74) Vertreter: Siemens Healthineers Patent Attorneys

(56) Entgegenhaltungen:
- CN-U- 206 044 918
- JP-A- 2013 022 246
- JP-A- 2014 061 111
- JP-A- 2019 115 382
- US-A1- 2010 134 315
- US-A1- 2016 360 987

## Beschreibung

Die vorliegende Erfindung betrifft eine Magnetresonanzvorrichtung mit einer Scannereinheit, einem bewegbaren Patiententisch, einer Patiententischsteuerung und einer Benutzerschnittstelle für eine Benutzereingabe zur Einstellung einer Bewegung des Patiententischs. Des Weiteren betrifft die vorliegende Erfindung auch ein Verfahren zu einem Steuern einer Bewegung eines Patiententischs einer Magnetresonanzvorrichtung. Zudem betrifft die Erfindung ein Computerprogrammprodukt, welches ein Programm umfasst zu einer Ausführung des Verfahrens zu einem Steuern einer Bewegung eines Patiententischs einer Magnetresonanzvorrichtung.

Bei Magnetresonanzuntersuchungen wird der Patient auf einem Patiententisch liegend in einen Patientenaufnahmebereich hineingefahren. Da am Patienten für die Magnetresonanzuntersuchung häufig Zubehöreinheiten angebracht sind, wie beispielsweise eine Injektionseinheit und/oder ein Atemsensor usw., ist eine Bewegung des Patiententisches mit besonderen Risiken behaftet, da hierbei Kabel und/oder Schläuche während einer Patiententischbewegung zwischen dem Patiententisch und einem den Patientenaufnahmebereich umgebenden Gehäuse eingeklemmt werden können. Dieses Risiko wird noch erhöht, wenn zusätzlich am Patienten eine Intervention durchgeführt wird und damit weitere Zusatzeinheiten für die Intervention auf dem Patiententisch bereitstehen. Von daher ist es wichtig, dass Fehlfunktionen des Patiententischs und damit unerwünschte Bewegungen des Patiententischs verhindert werden.

Verfahren zur Verhinderung solcher unerwünschter Bewegungen des Patiententischs sind beispielsweise in den Schriften JP2013022246A, JP2019115382A, US2010134315A1 und CN206044918U gezeigt.

Der vorliegenden Erfindung liegt insbesondere die Aufgabe zugrunde, eine unerwünschte Bewegung eines Patiententischs zu verhindern. Die Aufgabe wird durch die Merkmale der unabhängigen Ansprüche gelöst. Vorteilhafte Ausgestaltungen sind in den Unteransprüchen beschrieben.

Die Erfindung geht aus von einer Magnetresonanzvorrichtung mit einer Scannereinheit, einem bewegbaren Patiententisch, einer Patiententischsteuerung und einer Benutzerschnittstelle für eine Benutzereingabe zur Einstellung einer Bewegung des Patiententischs. Erfindungsgemäß umfasst die Patiententischsteuerung zumindest zwei Steuerpfade, wobei mittels der zumindest zwei Steuerpfade eine Benutzereingabe mittels der Benutzerschnittstelle erfassbar ist.

Die Scannereinheit der Magnetresonanzvorrichtung umfasst typischerweise einen Grundmagneten. Der Grundmagnet ist zu einem Erzeugen eines starken, homogenen und konstanten Grundmagnetfelds ausgelegt, wobei der Grundmagnet derart ausgebildet ist, dass das starke, homogene und konstante Grundmagnetfeld in einem Patientenaufnahmebereich, insbesondere in einem Field of View (FOV), der Magnetresonanzvorrichtung angelegt und/oder angeordnet ist. Des Weiteren umfasst die Scannereinheit bevorzugt eine Gradientenspuleneinheit. Die Gradientenspuleneinheit ist zu einer Erzeugung von Magnetfeldgradienten für eine Ortskodierung innerhalb des Patientenaufnahmebereichs, insbesondere innerhalb des FOVs, während einer Magnetresonanzbildgebung ausgelegt und/oder ausgebildet. Weiterhin umfasst die Scannereinheit bevorzugt eine Hochfrequenzantenneneinheit, wobei die Hochfrequenzantenneneinheit fest innerhalb der Scannereinheit integriert ist. Mittels der Hochfrequenzantenneneinheit können während einer Magnetresonanzuntersuchung vorteilhaft hochfrequente Magnetresonanzsequenzen generiert und in den Patientenaufnahmebereich, insbesondere in das FOV, zur Anregung ausgesendet und/oder ausgestrahlt werden.

Die Magnetresonanzvorrichtung umfasst weiterhin den Patientenaufnahmebereich, der zumindest teilweise von der Scannereinheit umgeben ist. Beispielsweise kann der Patientenaufnahmebereich zylinderförmig von der Scannereinheit umgeben sein. Der Patientenaufnahmebereich ist zur Aufnahme des Patienten und/oder eines zu untersuchenden Bereichs des Patienten während einer Magnetresonanzuntersuchung ausgelegt und/oder ausgebildet. Der Patientenaufnahmebereich umfasst bevorzugt das FOV und/oder das Isozentrum der Magnetresonanzvorrichtung. Das FOV umfasst bevorzugt einen Erfassungsbereichs der Magnetresonanzvorrichtung, innerhalb dessen Magnetresonanzdaten erfasst werden können. Das Isozentrum der Magnetresonanzvorrichtung umfasst bevorzugt den Bereich und/oder Punkt innerhalb der Magnetresonanzvorrichtung, der die optimalsten und/oder idealsten Bedingungen für eine Magnetresonanzuntersuchung aufweist. Insbesondere umfasst das Isozentrum den homogensten Bereich innerhalb der Magnetresonanzvorrichtung. Vorzugsweise ist das Isozentrum innerhalb des Patientenaufnahmebereichs angeordnet.

Innerhalb des Patientenaufnahmebereichs ist für gewöhnlich eine Patientenlagerungsvorrichtung bewegbar angeordnet. Insbesondere ist hierbei der Patiententisch der Patientenlagerungsvorrichtung in eine z-Richtung der Scannereinheit und/ oder in Richtung einer Längserstreckung des Patientenaufnahmebereichs bewegbar angeordnet. Zudem ist der Patiententisch auch in eine vertikale Richtung bewegbar angeordnet, um beispielsweise Patiententisch abzusenken und ein Aufsitzen eines Patienten auf den Patiententisch zu ermöglichen. Hierzu ist bevorzugt der Patiententisch komplett aus dem Patientenaufnahmebereich ausgefahren. Auf dem Patiententisch ist der Patient für die Magnetresonanzuntersuchung positioniert. Zudem sind mit dem Patienten zusammen noch Zubehöreinheiten, wie beispielsweis lokale Hochfrequenzantenneneinheiten und/oder Injektionseinheiten und/oder EKG-Einheiten usw. auf dem Patiententisch positioniert. Mittels des bewegbaren Patiententischs kann ein Patient, insbesondere ein zu untersuchender Bereich des Patienten, in den Patientenaufnahmebereich für eine Magnetresonanzuntersuchung eingebracht und/oder eingefahren werden und/oder für eine Magnetresonanzuntersuchung innerhalb des Patientenaufnahmebereichs, insbesondere innerhalb des FOVs, positioniert werden.

Die Benutzerschnittstelle für eine Benutzereingabe zur Einstellung einer Bewegung des Patiententischs ist bevorzugt an der Scannereinheit der Magnetresonanzvorrichtung angeordnet. Alternativ oder zusätzlich kann die Benutzerschnittstelle für eine Benutzereingabe zur Einstellung einer Bewegung des Patiententischs auch direkt an der Patientenlagerungsvorrichtung und/oder an dem bewegbaren Patiententisch angeordnet sein. Die Benutzerschnittstelle umfasst bevorzugt zumindest ein Eingabeelement und/oder Bedienelement, mittels dessen ein Benutzer, insbesondere ein medizinisches Bedienpersonal, die Benutzereingabe tätigen kann. Das zumindest eine Eingabeelement und/oder Bedienelement umfasst einen Drehschalter und kann weitere Elemente wie eine Taste und/oder einen Schalter und/oder eine Schaltfläche und/oder ein Touch-Display und/oder weitere, dem Fachmann als sinnvoll umfassende Bedienelemente umfassen. Die Benutzereingabe umfasst eine Eingabe, die an eine Patiententischsteuerung weitergeleitet wird und dort anhand der Eingabe ein Steuerbefehl für eine Bewegung des Patiententischs generiert wird.

Die Patiententischsteuerung umfasst bevorzugt eine Steuereinheit und eine Recheneinheit. Die Steuereinheit und Recheneinheit der Patiententischsteuerung ist zu einer Generierung eines Steuerbefehls zur Steuerung des Patiententischs ausgelegt und/oder ausgebildet. Die Steuereinheit und die Recheneinheit umfasst zumindest ein Rechenmodul und/oder einen Prozessor, wobei die Steuereinheit und die Recheneinheit zur Steuerung einer Bewegung des Patiententischs ausgelegt und/oder ausgebildet ist. So ist insbesondere die Steuereinheit und Recheneinheit dazu ausgebildet, computerlesbare Instruktionen auszuführen, um eine Bewegung des Patiententischs zu steuern. Insbesondere umfasst die Steuereinheit und/oder Recheneinheit eine Speichereinheit, wobei auf der Speichereinheit computerlesbare Informationen gespeichert sind, wobei die Steuereinheit und/oder Recheneinheit dazu ausgebildet ist, die computerlesbaren Informationen von der Speichereinheit zu laden und die computerlesbaren Informationen auszuführen, um eine Bewegung des Patiententischs zu steuern.

Die Komponenten der Steuereinheit und/oder Recheneinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Die Steuereinheit und/oder Recheneinheit kann dabei innerhalb einer Systemsteuereinheit zur Steuerung der Magnetresonanzvorrichtung integriert und/oder angeordnet sein. Bevorzugt umfasst die Steuereinheit und/oder Recheneinheit der Patiententischsteuerung eine zur Systemsteuereinheit der Magnetresonanzvorrichtung separate Einheit. Hierbei kann die Steuereinheit und/oder Recheneinheit der Patiententischsteuerung mittels einer Datenaustauscheinheit zwecks eines Informationsaustauschs und/oder eines Datenaustauschs mit der Systemsteuereinheit verbunden sein. Die Datenaustauscheinheit kann hierbei zu einem kabellosen Datenaustausch und/oder einem kabelgebundenen Datenaustausch zwischen der Systemsteuereinheit und der Steuereinheit und/oder Recheneinheit der Patiententischsteuerung ausgelegt und/oder ausgebildet sein.

Mittels der Patiententischsteuerung werden für eine Bewegung des Patiententischs Steuerbefehle und/oder Steuersignale generiert. Die Generierung der Steuerbefehle und/oder Steuersignale zur Bewegung des Patiententischs ist dabei abhängig von der Benutzereingabe. Zur Erfassung der der Benutzereingabe weist die Patiententischsteuerung zwei Steuerpfade auf. Jeder er zumindest zwei Steuerpfade ist zu einer Erfassung der Benutzereingabe mittels der Benutzerschnittstelle, mittels des Eingabeelements und/oder des Bedienelements der Benutzerschnittstelle, ausgelegt und/oder ausgebildet. Die einzelnen Steuerpfade können hierbei auch weiter Steuerelemente und/oder Schaltelemente und/oder Daten- übertragungselemente umfassen, um die Benutzereingabe mittels der Benutzerschnittstelle, insbesondere mittels des Eingabeelements und/oder des Bedienelements der Benutzerschnittstelle, zu erfassen und an die Steuereinheit und/oder Recheneinheit der Patiententischsteuerung weiterzuleiten.

Durch die Erfindung wird eine redundante Erfassung der mittels der Benutzerschnittstelle, d. h. mittels des Eingabeelements und/oder des Bedienelements der Benutzerschnittstelle, getätigten Benutzereingabe erreicht. Damit einhergehend kann eine Sicherheit und/oder Zuverlässigkeit bei einer Steuerung einer Bewegung des Patiententischs erhöht werden, insbesondere können derart unerwünschte Bewegung des Patiententischs verhindert werden. Zudem kann derart eine hohe Patientensicherheit erreicht werden und Verletzungen des Patienten aufgrund eines unerwünschten Bewegens des Patiententischs aufgrund einer Fehlsteuerung vorteilhaft verhindert werden.

In der erfindungsgemäßen Magnetresonanzvorrichtung ist vorgesehen , dass die Benutzerschnittstelle ein Bedienelement aufweist, das einen Drehschalter umfasst. Das Bedienelement ist besonders vorteilhaft zu einer Bedienung des Patiententischs, insbesondere zu einer Einstellung einer Position des Patiententischs durch einen Benutzer, insbesondere in medizinisches Bedienpersonal, ausgelegt und/oder ausgebildet. Hierbei kann das Bedienelement ausschließlich zur Bedienung des Patiententischs ausgelegt und/oder ausgebildet sein. Besonders vorteilhaft ist hierbei das Bedienelement an der Scannereinheit der Magnetresonanzvorrichtung, insbesondere an einer Frontseite der Scannereinheit, angeordnet, so dass eine vorteilhafte Erreichbarkeit während einer Patientenvorbereitung für einen Benutzer,

insbesondere einem medizinischen Bedienpersonal, gegeben ist. Das Bedienelement ist als Drehschalter ausgebildet. Der Drehschalter kann dabei einen Drehknopf und/oder eine Drehtaste umfassen, wobei der Drehschalter derart ausgebildet ist, dass durch Drehen des Drehschalters eine Eingabe getätigt werden kann. Mittels des Drehschalters kann besonders einfach eine Bewegung des Patiententischs durch ein manuelles Drehen des Drehschalters eingestellt werden. Dabei kann beispielsweise auch eine Bewegungsrichtung durch Auswahl einer Drehrichtung des Drehschalters von dem Bediener eingestellt werden. Insbesondere kann derart eine besonders einfache Bedienung des Patiententischs und damit auch eine besonders einfache Einstellung einer Bewegung des Patiententischs durch einen Benutzer, insbesondere ein medizinisches Bedienpersonal, vorteilhaft erreicht werden.

In einer vorteilhaften Weiterbildung der erfindungsgemäßen Magnetresonanzvorrichtung kann es vorgesehen sein, dass die Benutzerschnittstelle ein Bedienelement aufweist, das einen druckempfindlichen Drehschalter umfasst. Vorzugsweise ist das Bedienelement als druckempfindlicher Drehschalter ausgebildet. Der druckempfindliche Drehschalter kann dabei einen druckempfindlichen Drehknopf und/oder eine druckempfindliche Drehtaste umfassen, wobei der druckempfindliche Drehschalter derart ausgebildet ist, dass sowohl durch Drehen des druckempfindlichen Drehschalters als auch durch Drücken des druckempfindlichen Drehschalters eine Eingabe getätigt werden kann. Insbesondere kann derart eine besonders einfache Bedienung des Patiententischs und damit auch eine besonders einfache Einstellung einer Bewegung des Patiententischs durch einen Benutzer, insbesondere ein medizinisches Bedienpersonal, vorteilhaft erreicht werden. Des Weiteren kann derart mittels eines einzigen Bedienelements eine Eingabe unterschiedlicher Einstellmöglichkeiten zur Steuerung des Patiententischs für einen Benutzer, insbesondere ein medizinisches Bedienpersonal, bereitgestellt werden und damit eine hoher Bedienkomfort für den Benutzer, insbesondere ein medizinisches Bedienpersonal, erreicht werden.

In der erfindungsgemäßen Magnetresonanzvorrichtung ist es vorgesehen, dass die Benutzerschnittstelle ein Bedienelement aufweist, wobei eine Erfassung einer Benutzereingabe an dem Bedienelement mittels des ersten Steuerpfads der beiden Steuerpfade unabhängig von einer Erfassung der Benutzereingabe an dem Bedienelement mittels des zweiten Steuerpfads der beiden Steuerpfade ist. Insbesondere weist hierbei der erste Steuerpfad eine Steuerelektronik auf, die unabhängig und/oder separat zur einer Steuerelektronik des zweiten Steuerpfads ausgebildet ist. Besonders vorteilhaft sind hierbei der erste Steuerpfad und der zweiten Steuerpfad parallel zueinander ausgebildet, wobei die Patiententischsteuerung eine Parallelschaltung aufweist, die den ersten Steuerpfad und den zweiten Steuerpfad umfasst. Vorzugsweise ist das Bedienelement, beispielsweise ein druckempfindlicher Drehschalter, mit den beiden Steuerpfaden der Patiententischsteuerung gekoppelt. Dies ermöglicht vorteilhaft eine redundante Erfassung einer Benutzereingabe, so dass eine Fehlsteuerung des Patiententischs, beispielsweise aufgrund einer fehlerhaften Erfassung der Benutzereingabe, verhindert werden kann.

In der erfindungsgemäßen Magnetresonanzvorrichtung ist es vorgesehen, dass die Patiententischsteuerung eine Recheneinheit aufweist und die Recheneinheit zu Generierung eines Steuerbefehls für den Patiententisch ausgelegt ist, wobei die Recheneinheit zwei separate Eingänge aufweist und jeder der beiden Eingänge einem der beiden Steuerpfade zugeordnet ist. Derart können die beiden unabhängig und/oder separat erfassten Benutzereingaben, die mittels der Benutzerschnittstelle eingegeben werden, zur Steuerung und/oder Bedienung des Patiententischs unabhängig und/oder separat voneinander von der Recheneinheit zur Generierung eines Steuerbefehls verarbeitet werden. Die Recheneinheit ist dazu ausgelegt und/oder ausgebildet, einen Steuerbefehl zu generieren, wenn die mittels der beiden unabhängigen Steuerpfade erfassten und übermittelten Benutzerinformationen und/oder Eingabeinformationen eine gleiche Information und/oder eine übereinstimmende Information und/oder eine korrespondierende Information umfassen. Liegt beispielsweise aus beiden Steuerpfaden eine Information für ein Einfahren des Patiententischs in den Patientenaufnahmebereich vor, wird von Recheneinheit ein entsprechender Steuerbefehl generiert und an den Patiententisch weitergegeben. Liegen dagegen widersprüchliche Informationen durch die beiden Steuerpfade hinsichtlich einer Bewegung des Patiententischs in der Recheneinheit vor, wird von der Recheneinheit kein Steuerbefehl zur Bewegung des Patiententischs generiert. Beispielsweise kann dabei nur ein Steuerpfad der beiden Steuerpfade eine Information für eine Bewegung des Patiententischs umfassen, während bei dem zweite Steuerpfad keine Informationen für eine Bewegung des Patiententischs vorliegen. Dabei kann es auch sein, dass bei Vorliegen von widersprüchlichen Informationen durch die beiden Steuerpfade hinsichtlich einer Bewegung des Patiententischs in der Recheneinheit von der Recheneinheit ein Stoppbefehl und/oder ein Arretierbefehl generiert wird, der eine Bewegung des Patiententischs verhindert und/oder blockiert und/oder eine aktuelle Bewegung des Patiententischs stoppt.

In einer vorteilhaften Weiterbildung der erfindungsgemäßen Magnetresonanzvorrichtung kann es vorgesehen sein, dass zumindest ein Steuerpfad der beiden Steuerpfade eine Schalteinheit mit einer Anzahl an Schaltelementen umfasst, wobei die Anzahl der Schaltelemente abhängig ist von einer Anzahl an unterschiedlichen Einstellpositionen eines Bedienelements der Benutzerschnittstelle. Die Schalteinheit ist dazu ausgelegt und/oder ausgebildet, eine Einstellposition des Bedienelements zur erfassen. Eine Einstellposition des Bedienelements kann beispielsweise eine Drehrichtung des Bedienelements und/oder eine gedrückte Position des Bedienelements usw. umfassen. Bevorzugt steht hierbei für jede Einstellposition des Bedienelements ein Schaltelement in der Schalteinheit zur Verfügung, so dass jede der Einstellpositionen unabhängig erfasst werden kann. Die einzelnen Schaltelemente umfassen bevorzugt elektronische Schaltelemente. Besonders vorteilhaft umfasst jeder Steuerpfad der beiden Steuerpfade eine Schalteinheit mit einer Anzahl an Schaltelementen, wobei die Anzahl der Schaltelemente abhängig ist von einer Anzahl an unterschiedlichen Einstellpositionen des Bedienelements der Benutzerschnittstelle. Diese Ausgestaltung der Erfindung ermöglicht eine besonders zuverlässige Erfassung einer Einstellung einer Position des Bedienelements. Damit kann auch eine unerwünschte Fehlsteuerung des Patiententisch reduziert und/oder verhindert werden.

In einer vorteilhaften Weiterbildung der erfindungsgemäßen Magnetresonanzvorrichtung kann es vorgesehen sein, dass die Schalteinheit drei Schaltelemente aufweist. Derart kann besonders vorteilhaft eine Einstellposition eines Bedienelements, insbesondere eines druckempfindlichen Drehschalters, vorteilhaft erfasst werden.

In einer vorteilhaften Weiterbildung der erfindungsgemäßen Magnetresonanzvorrichtung kann es vorgesehen sein, dass zumindest einer der beiden Steuerpfade ein Intensitätsmesseinheit zur Erfassung einer Intensität einer Eingabe an einem Bedienelement aufweist. Die Intensität einer Eingabe an einem Bedienelement kann beispielsweise einen Grad und/oder einen Umfang einer Drehbewegung eines Drehschalters umfassen. Zudem kann die Intensität einer Eingabe an einem Bedienelement auch eine Zeit einer Drückbewegung einer Drucktaste und/oder eine zurückgelegte Strecke einer Drucktaste und/oder eine zurückgelegte Strecke eines Schiebereglers usw. umfassen. Das Intensitätsmesseinheit kann dabei ein Potentiometer und/oder weitere, dem Fachmann als sinnvoll erscheinende Messeinheiten zur Erfassung einer Intensität einer Eingabe an einem Bedienelement durch einen Benutzer umfassen. Hierdurch kann vorteilhaft ein Steuergröße abhängig von der erfassten Intensität eingestellt und/oder bestimmt werden.

In einer vorteilhaften Weiterbildung der erfindungsgemäßen Magnetresonanzvorrichtung kann es vorgesehen sein, dass mittels der Patiententischsteuerung zumindest ein Bewegungsparameter zur Steuerung des Patiententischs in Abhängigkeit der erfassten Intensität der mittels des Bedienelements getätigten Eingabe einstellbar ist. Der zumindest eine Bewegungsparameter kann dabei ein Parameter einer zurückzulegenden Strecke des Patiententischs umfassen. Erfindungsgemäß umfasst der zumindest eine Bewegungsparameter einen Parameter einer Geschwindigkeit einer Bewegung des Patiententischs. Derart wird anhand eines Umfangs einer Drehbewegung eines Drehschalters eine Geschwindigkeit für eine Einfahrbewegung und/oder eine Ausfahrbewegung des Patiententischs bestimmt . Derart kann mittels eines einzigen Bedienelements, beispielsweise mittels eines druckempfindlichen Drehschalters, eine Eingabe unterschiedlicher Einstellmöglichkeiten zur Steuerung des Patiententischs für einen Benutzer, insbesondere ein medizinisches Bedienpersonal, bereitgestellt werden. Zusätzlich kann hierdurch ein hoher Bedienkomfort für den Benutzer, insbesondere ein medizinisches Bedienpersonal, bereitgestellt werden.

In einer außerhalb des Schutzumfangs der Erfindung liegenden Magnetresonanzvorrichtung kann es vorgesehen sein, dass die Benutzerschnittstelle ein erstes Bedienelement und ein zweiten Bedienelement aufweist, wobei der erste Steuerpfad der beiden Steuerpfade mit dem ersten Bedienelement gekoppelt ist und der zweite Steuerpfad der beiden Steuerpfade mit dem zweiten Bedienelement gekoppelt ist. Für eine Generierung eines Steuerbefehls mittels einer Recheneinheit der Patiententischsteuerung liegen bevorzugt korrespondierende und/oder übereinstimmende Informationen, die von den beiden Steuerpfaden von den beiden Bedienelementen an die Recheneinheit übertragen werden, in der Recheneinheit vor. Korrespondierende und/oder übereinstimmende Informationen liegen dann vor, wenn durch das erste Bedienelement eine Bewegungsinformation, beispielsweise ein Einführen des Patiententischs in den Patientenaufnahmebereich, vorliegt und auch durch das zweite Bedienelement eine Bewegungsinformation, beispielsweise ein Einführen des Patiententischs in den Patientenaufnahmebereich, vorliegt. Zudem können eine korrespondierende und/oder übereinstimmende Informationen vorliegen, wenn durch das erste Bedienelement eine Bewegungsinformation vorliegt und durch das zweite Bedienelement eine Freigabe für eine Bewegung vorliegt. Das erste Bedienelement und das zweite Bedienelement können dabei gleichartig ausgebildet sein. Zudem kann das erste Bedienelement auch unterschiedlich zu dem zweiten Bedienelement ausgebildet sein. Hierdurch kann vorteilhaft eine redundante Erfassung der Benutzereingabe mittels der beiden Bedienelemente erreicht werden. Zudem kann eine Sicherheit und/oder Zuverlässigkeit bei der Steuerung einer Bewegung des Patiententischs erhöht werden, insbesondere können derart unerwünschte Bewegung des Patiententischs verhindert werden.

In dieser Magnetresonanzvorrichtung kann es vorgesehen sein, dass das erste Bedienelement und/oder das zweite Bedienelement eine Arretierfunktion des Patiententischs aufweist. Vorzugsweise umfasst die Arretierfunktion des ersten Bedienelements und/oder des zweiten Bedienelements einer elektronische Arretierung und/oder Verriegelung des Patiententischs, so dass kein Steuerbefehl für eine Bewegung des Patiententischs zum Patiententisch gelangen kann und/oder kein Steuerbefehl für eine Bewegung des Patiententischs in der Recheneinheit generiert wird. Diese Ausgestaltung ermöglicht einen hohen Sicherheitsstandard bei einem Betrieb der Magnetresonanzvorrichtung, insbesondere des Patiententischs der Magnetresonanzvorrichtung. Insbesondere kann derart während einer Aktivierung der Arretierfunktion, beispielsweise durch Drücken des ersten Bedienelements und/oder des zweiten Bedienelements, ein unbeabsichtigtes Bewegen des Patiententischs vorteilhaft verhindert werden. Zudem kann eine hohe Patientensicherheit erreicht werden und Verletzungen des Patienten aufgrund eines unerwünschten Bewegens des Patiententischs vorteilhaft verhindert werden.

Des Weiteren geht die Erfindung aus von einem Verfahren zu einem Steuern einer Bewegung eines Patiententischs der erfindungsgemäßen Mag-netresonanzvorrichtung, wobei das Verfahren die folgenden Verfahrensschritte umfasst:
- Erfassen von ersten Eingabedaten einer Eingabe an zumindest einem Bedienelement mittels eines ersten Steuerpfads,
- Erfassen von zweiten Eingabedaten einer Eingabe an dem zumindest einen Bedienelement mittels eines zweiten Steuerpfads,
- Übertragen der ersten Eingabedaten und der zweiten Eingabedaten an eine Recheneinheit,
- Auswerten der ersten Eingabedaten und der zweiten Eingabedaten mittels der Recheneinheit und Generieren eines Steuerbefehls zur Steuerung des Patiententischs, wobei das Generieren des Steuerbefehls mittels der Recheneinheit erfolgt, sobald die ersten Eingabedaten und die zweiten Eingabedaten eine übereinstimmende Eingabeinformation umfassen, und
- Ausführen des Steuerbefehls zur Steuerung der Bewegung des Patiententischs.

Vorzugsweise umfassen die ersten Eingabedaten und/oder die zweiten Eingabedaten Benutzereingabedaten, die manuell von einem Benutzer, insbesondere dem medizinischen Bedienpersonal, mittels des zumindest einen Bedienelements eingegeben werden. Das zumindest eine Bedienelement umfasst einen Drehschalter zur Benutzereingabe für eine Steuerung und/oder Bewegung des Patiententischs. Die Recheneinheit ist dazu ausgelegt und/oder ausgebildet, einen Steuerbefehl zu generieren, wenn die mittels der beiden Steuerpfade erfassten und übermittelten Eingabeinformationen eine gleiche Information und/oder eine übereinstimmende Information und/oder eine korrespondierende Information umfassen. Liegt beispielsweise aus beiden Steuerpfaden eine Eingabeinformation für ein Einfahren des Patiententischs in den Patientenaufnahmebereich vor, wird von Recheneinheit ein entsprechender Steuerbefehl generiert und an den Patiententisch weitergegeben. Liegen dagegen widersprüchliche Einageinformationen durch die beiden Steuerpfade hinsichtlich einer Bewegung des Patiententischs in der Recheneinheit vor, wird von der Recheneinheit kein Steuerbefehl zur Bewegung des Patiententischs generiert.

Durch das erfindungsgemäße Verfahren kann vorteilhaft eine redundante Erfassung der mittels der Benutzerschnittstelle, insbesondere mittels des Eingabeelements und/oder des Bedienelements der Benutzerschnittstelle, getätigten Benutzereingabe erreicht werden. Damit einhergehend kann eine Sicherheit und/oder Zuverlässigkeit bei einer Steuerung einer Bewegung des Patiententischs erhöht werden, insbesondere können derart unerwünschte Bewegung des Patiententischs verhindert werden. Zudem kann derart eine hohe Patientensicherheit erreicht werden und Verletzungen des Patienten aufgrund eines unerwünschten Bewegens des Patiententischs aufgrund einer Fehlsteuerung vorteilhaft verhindert werden.

Die Vorteile des erfindungsgemäßen Verfahrens zu einem Steuern einer Bewegung eines Patiententischs einer Magnetresonanzvorrichtung entsprechen im Wesentlichen den Vorteilen der erfindungsgemäßen Magnetresonanzvorrichtung, welche vorab im Detail ausgeführt sind. Hierbei erwähnte Merkmale, Vorteile oder alternative Ausführungsformen können auch auf die anderen beanspruchten Gegenstände übertragen werden und umgekehrt.

Im erfindungsgemäßen Verfahren ist es vorgesehen, dass das Erfassen der ersten Eingabedaten mittels des ersten Steuerpfads unabhängig von dem Erfassen der zweiten Eingabedaten mittels des zweiten Steuerpfads ist. Dabei ist auch der erste Steuerpfad unabhängig von dem zweiten Steuerpfad ausgebildet, so dass eine unabhängige Erfassung von Eingabedaten und auch eine unabhängige Übertragung der erfassten Eingabedaten mittels der beiden Steuerpfade erfolgen kann. Insbesondere weist hierbei der erste Steuerpfad eine Steuerelektronik auf, die unabhängig und/oder separat zur einer Steuerelektronik des zweiten Steuerpfads ausgebildet ist. Besonders vorteilhaft sind hierbei der erste Steuerpfad und der zweiten Steuerpfad parallel zueinander ausgebildet, wobei die Patiententischsteuerung eine Parallelschaltung aufweist, die den ersten Steuerpfad und den zweiten Steuerpfad umfasst. Das Bedienelement umfasst einen Drehschalter, beispielsweise einen druckempfindlicher Drehschalter, und ist mit den beiden Steuerpfaden der Patiententischsteuerung gekoppelt. Dies ermöglicht vorteilhaft eine redundante Erfassung einer Benutzereingabe, so dass eine unerwünschte Fehlsteuerung des Patiententischs, beispielsweise aufgrund einer fehlerhaften Erfassung der Benutzereingabe, vorteilhaft verhindert werden kann.

In einer vorteilhaften Weiterbildung des erfindungsgemäßen Verfahrens kann es vorgesehen sein, dass die Eingabe an dem zumindest einen Bedienelement eine Drehbewegung und/oder eine Drückbewegung an einem druckempfindlichen Drehschalter umfasst. Der druckempfindliche Drehschalter kann dabei einen druckempfindlichen Drehknopf und/oder eine druckempfindliche Drehtaste umfassen, wobei der druckempfindliche Drehschalter derart ausgebildet ist, dass sowohl durch Drehen des druckempfindlichen Drehschalters als auch durch Drücken des druckempfindlichen Drehschalters eine Eingabe getätigt werden kann. Insbesondere kann derart eine besonders einfache Bedienung des Patiententischs und damit auch eine besonders einfache Einstellung einer Bewegung des Patiententischs durch einen Benutzer, insbesondere ein medizinisches Bedienpersonal, vorteilhaft erreicht werden. Des Weiteren kann derart mittels eines einzigen Bedienelements eine Eingabe unterschiedlicher Einstellmöglichkeiten zur Steuerung des Patiententischs für einen Benutzer, insbesondere ein medizinisches Bedienpersonal, bereitgestellt werden und damit eine hoher Bedienkomfort für den Benutzer, insbesondere ein medizinisches Bedienpersonal, erreicht werden.

In einer vorteilhaften Weiterbildung des erfindungsgemäßen Verfahrens kann es vorgesehen sein, dass in der Recheneinheit zumindest ein aktueller Zustandsparameter des Patiententischs vorliegt und der Steuerbefehl in Abhängigkeit von dem zumindest einen Zustandsparameter des Patiententischs von der Recheneinheit generiert wird. Hierdurch kann eine einfache und insbesondere kontextabhängige Steuerung und/oder Einstellung des Patiententischs erreicht werden. Damit einhergehend kann eine hoher Bedienkomfort für einen Benutzer, insbesondere ein medizinisches Bedienpersonal, vorteilhaft bereitgestellt werden.

Der zumindest eine aktuelle Zustandsparameter des Patiententischs kann eine aktuelle Position des Patiententischs umfassen. Die aktuelle Position des Patiententischs kann beispielsweise eine Home-Position des Patiententischs umfassen, wobei in der Home-Position der Patiententisch bereit für eine Einfahren und/oder Einführen in den Patientenaufnahmebereich ist. Des Weiteren kann die aktuelle Position des Patiententischs beispielswiese eine Isozentrums-Position umfassen, wobei in der Isozentrums-Position der Patiententisch, insbesondere ein zu untersuchender Bereich des Patienten, im Isozentrum der Magnetresonanzvorrichtung angeordnet ist. Des Weiteren kann die aktuelle Position des Patiententischs beispielswiese eine abgesenkte Position des Patiententischs umfassen, wie dies insbesondere zu einer Positionierung und/oder Lagerung des Patienten von Vorteil ist.

Zudem kann der zumindest eine aktuelle Zustandsparameter des Patiententischs einen aktuelle Bewegungszustand umfassen. Der aktuelle Bewegungsstand des Patiententischs kann dabei ein Stillstehen des Patiententischs umfassen oder auch eine aktuelle Geschwindigkeit des Patiententischs, mit der der Patiententisch gerade bewegt wird. Des Weiteren kann der zumindest eine aktuelle Zustandsparameter des Patiententischs auch eine aktuelle Zielposition umfassen. Die aktuelle Zielposition beispielsweise die Home-Position des Patiententischs und/oder die Isozentrums-Position des Patiententischs und/oder eine abgesenkte Position des Patiententischs umfassen. Zudem kann der zumindest eine aktuelle Zustandsparameter des Patiententischs weitere, dem Fachmann als sinnvoll erscheinende Zustandsparameter des Patiententischs umfassen. Der zumindest eine aktuelle Zustandsparameter des Patiententischs kann dabei in der Recheneinheit gespeichert sein. Zudem kann die Magnetresonanzvorrichtung eine Erfassungseinheit zur Erfassung des zumindest einen aktuellen Zustandsparameters des Patiententischs aufweisen, wie beispielsweise eines Sensoreinheit.

Befindet sich beispielsweise der Patiententisch in einer abgesenkten Position, beispielsweise für eine Patientenvorbereitung, kann ein Drücken des Bedienelements ein Anheben des Patiententisch bewirken, um den Patiententisch in einer Home-Position für ein Einfahren in den Patientenaufnahmebereich zu positionieren. Befindet sich beispielsweise der Patiententisch bereits in der Home-Position, kann ein Drücken des Bedienelements ein Einfahren des Patiententischs in eine Isozentrum-Position bewirken, so dass der Patiententisch in einer Untersuchungsposition innerhalb des Patientenaufnahmebereich positioniert ist.

In einer vorteilhaften Weiterbildung des erfindungsgemäßen Verfahrens kann es vorgesehen sein, dass der generiert Steuerbefehl eine Richtung einer Bewegung des Patiententischs umfasst, wobei die Richtung der Bewegung des Patiententischs abhängig von einer Drehrichtung des Drehschalters ist. Hierdurch kann eine einfache und intuitive Bedienung und/oder Einstellung zur Steuerung und/oder Einstellung einer Bewegung des Patiententischs an dem Bedienelement für einen Benutzer, insbesondere ein medizinisches Bedienpersonal, vorteilhaft erreicht werden. Zudem kann durch eine derartige Ausgestaltung der Erfindung ein hoher Bedienkomfort für einen Benutzer, insbesondere ein medizinisches Bedienpersonal, bereitgestellt werden.

In einer vorteilhaften Weiterbildung des erfindungsgemäßen Verfahrens kann es vorgesehen sein, dass die Eingabedaten einer Eingabe an dem zumindest einen Bedienelement eine Arretierinformation für den Patiententisch umfassen. Die Arretierinformation kann besonders vorteilhaft eine elektronische Arretierung und/oder Verriegelung des Patiententischs umfassen, so dass kein Steuerbefehl für eine Bewegung des Patiententischs zum Patiententisch gelangen kann und/oder kein Steuerbefehl für eine Bewegung des Patiententischs in der Recheneinheit generiert wird. Derart kann vorteilhaft eine hohe Sicherheit und/oder Zuverlässigkeit bei der Bedienung und/oder Einstellung des Patiententischs bereitgestellt werden. Insbesondere kann derart eine Fehlsteuerung, die zu einem unerwünschten Bewegen des Patiententischs führen kann, vorteilhaft verhindert werden.

Des Weiteren geht die Erfindung aus von einem Computerprogrammprodukt, welches ein Programm umfasst und direkt in einem Speicher einer programmierbaren Recheneinheit ladbar ist, mit Programmmitteln, um ein Verfahren zu einem Steuern einer Bewegung eines Patiententischs einer Magnetresonanzvorrichtung auszuführen, wenn das Programm in der Recheneinheit ausgeführt wird. Dabei benötigt das Computerprogramm eventuell Programmmittel, **z.B.** Bibliotheken und Hilfsfunktionen, um die entsprechenden Ausführungsformen des Verfahrens zu realisieren. Das Computerprogramm kann dabei eine Software mit einen Quellcode, der noch kompiliert und gebunden oder der nur interpretiert werden muss, oder einen ausführbaren Softwarecode umfassen, der zur Ausführung nur noch in eine entsprechende Recheneinheit zu laden ist.

Das erfindungsgemäße Computerprogrammprodukt ist direkt in einen Speicher einer programmierbaren Recheneinheit ladbar und weist Programmcode-Mittel auf, um ein erfindungsgemäßes Verfahren auszuführen, wenn das Computerprogrammprodukt in der Recheneinheit ausgeführt wird. Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist derart konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Recheneinheit geladen werden kann, der mit der Magnetresonanzvorrichtung direkt verbunden oder als Teil ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuerung und/oder Recheneinheit gespeichert werden, können alle erfindungsgemäßen Ausführungsformen der vorab beschriebenen Verfahrens durchgeführt werden. So kann die Erfindung auch von dem besagten computerlesbaren Medium und/oder dem besagten elektronisch lesbaren Datenträger ausgehen.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäße Magnetresonanzvorrichtung in einer schematischen Darstellung
- Fig. 2: eine Übersichtsdarstellung einer Patiententischsteuerung,
- Fig. 3: eine Ansicht eines Bedienelements der Patiententischsteuerung,
- Fig. 4: eine Ansicht eines erfindungsgemäßen Verfahrens zu einem Steuern einer Bewegung eines Patiententischs einer Magnetresonanzvorrichtung, und
- Fig. 5: eine alternative Ausgestaltung der Patiententischsteuerung mit zwei Bedienelementen,
die außerhalb des Schutzumfangs der Erfindung liegt.

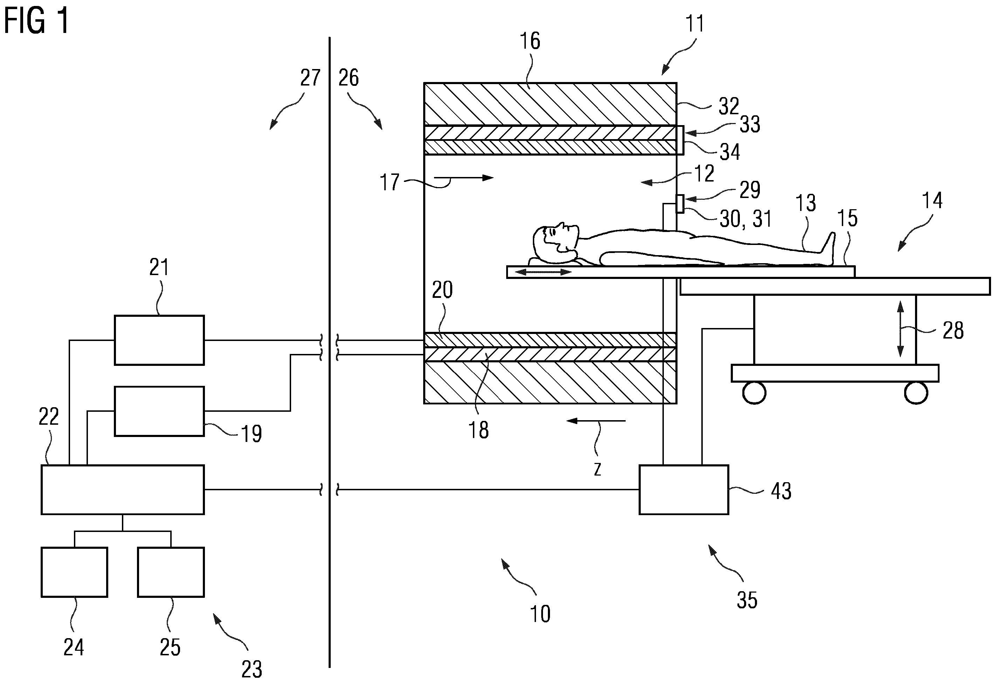

In der Fig. 1 ist eine Magnetresonanzvorrichtung 10 schematisch darstellt. Die Magnetresonanzvorrichtung 10 umfasst eine von einer Magneteinheit gebildeten Scannereinheit 11. Zudem weist die Magnetresonanzvorrichtung 10 einen Patientenaufnahmebereich 12 auf zu einer Aufnahme eines Patienten 13. Der Patientenaufnahmebereich 12 im vorliegenden Ausführungsbeispiel ist zylinderförmig ausgebildet und in einer Umfangsrichtung von der Scannereinheit 11, insbesondere von der Magneteinheit, zylinderförmig umgeben. Grundsätzlich ist jedoch eine davon abweichende Ausbildung des Patientenaufnahmebereichs 12 jederzeit denkbar.

Der Patient 13 kann mittels einer Patientenlagerungsvorrichtung 14 der Magnetresonanzvorrichtung 10 in den Patientenaufnahmebereich 12 geschoben und/oder gefahren werden. Die Patientenlagerungsvorrichtung 14 weist hierzu einen bewegbar ausgestalteten Patiententisch 15 auf. Insbesondere ist hierbei der Patiententisch 15 in Richtung einer Längserstreckung des Patientenaufnahmebereichs 12 und/oder in z-Richtung bewegbar gelagert für ein Einfahren des Patiententischs 15 in den Patientenaufnahmebereich 12. Zudem ist der Patiententisch 15 in vertikaler Richtung 28 einstellbar, um ein Aufsitzen des Patienten 13 auf den Patiententisch 15 zu erleichtern.

Die Scannereinheit 11, insbesondere die Magneteinheit, umfasst einen supraleitenden Grundmagneten 16 zu einem Erzeugen eines starken und insbesondere konstanten Grundmagnetfelds 17. Weiterhin weist die Scannereinheit 11, insbesondere die Magneteinheit, eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Scannereinheit 11, insbesondere die Magneteinheit, umfasst weiterhin eine Hochfrequenzantenneneinheit 20 zu einer Anregung einer Polarisation, die sich in dem von dem Grundmagneten 16 erzeugten Grundmagnetfeld 17 einstellt. Die Hochfrequenzantenneneinheit 20 wird von einer Hochfrequenzantennensteuereinheit 21 der Magnetresonanzvorrichtung 10 gesteuert und strahlt hochfrequente Magnetresonanzsequenzen in den Patientenaufnahmebereich 12 der Magnetresonanzvorrichtung 10 ein.

Zu einer Steuerung des Grundmagneten 16, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzbilder können auf einer Ausgabeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

Die Scannereinheit 11 der Magnetresonanzvorrichtung 10 ist zusammen mit der Patientenlagerungsvorrichtung 14 innerhalb eines Untersuchungsraums 26 angeordnet. Die Systemsteuereinheit 22 dagegen ist zusammen mit der Eingabeeinheit 25 und der Ausgabeeinheit 24 der Benutzerschnittstelle 23 innerhalb eines Kontrollraums 27 angeordnet. Der Kontrollraum 27 ist getrennt von dem Untersuchungsraum 26 ausgebildet. Insbesondere ist der Untersuchungsraum 26 hinsichtlich einer Hochfrequenzstrahlung von dem Kontrollraum 27 abgeschirmt.

Für eine Bedienung der Patientenlagerungsvorrichtung 14, insbesondere des Patiententischs 15, weist die Benutzerschnittstelle 23 eine weitere Eingabeeinheit 29 mit zumindest einem Bedienelement 30 und/oder Eingabeelement auf. Im vorliegenden Ausführungsbeispiel weist die weitere Eingabeeinheit 29 ein einziges Bedienelement 30 und/oder Eingabeelement auf, das einen Drehschalter 31, insbesondere einen druckempfindlichen Drehschalter 31, umfasst (Fig. 1 bis 3). Mittels des druckempfindlichen Drehschalters 31 kann sowohl eine Benutzereingabe durch ein Drücken P des druckempfindlichen Drehschalters 31 als auch durch ein Rotieren und/oder Drehen R_{R}, R_{L} des druckempfindlichen Drehschalters 31 ermöglicht werden. Die weitere Eingabeeinheit 29, insbesondere der druckempfindliche Drehschalter 31, ist innerhalb des Untersuchungsraums 26 angeordnet. Hierbei ist die weitere Eingabeeinheit 29, insbesondere der druckempfindliche Drehschalter 31, an der Scannereinheit 11 angeordnet. Für eine gute Erreichbarkeit ist die weitere Eingabeeinheit 29, insbesondere der druckempfindliche Drehschalter 31, an einer Frontseite 32 der Scannereinheit 11 angeordnet. Zudem weist im vorliegenden Ausführungsbeispiel die Benutzerschnittstelle eine weitere Ausgabeeinheit 33, die ein Display 34 und/oder eine Touch-Display umfasst. Die weitere Ausgabeeinheit 33, insbesondere das Display 34 und/oder das Touch-Display, ist ebenfalls an der Frontseite 32 der Scannereinheit 11 angeordnet. Insbesondere ist hierbei die weitere Ausgabeeinheit 33 in vertikaler Richtung 28 über der weiteren Eingabeeinheit 29 angeordnet, um ein Feedback für eine Eingabe direkt dem Benutzer, insbesondere dem medizinischen Bedienpersonal, mitzuteilen.

In einer alternativen Ausgestaltung der Benutzerschnittstelle 23 kann die weitere Eingabeeinheit 29, insbesondere der druckempfindliche Drehschalter 31, auch an dem Patiententisch 15 und/oder der Patientenlagerungsvorrichtung 14 angeordnet sein. Zudem kann auch die weitere Ausgabeeinheit 33 an dem Patiententisch 15 und/oder der Patientenlagerungsvorrichtung 14 angeordnet sein.

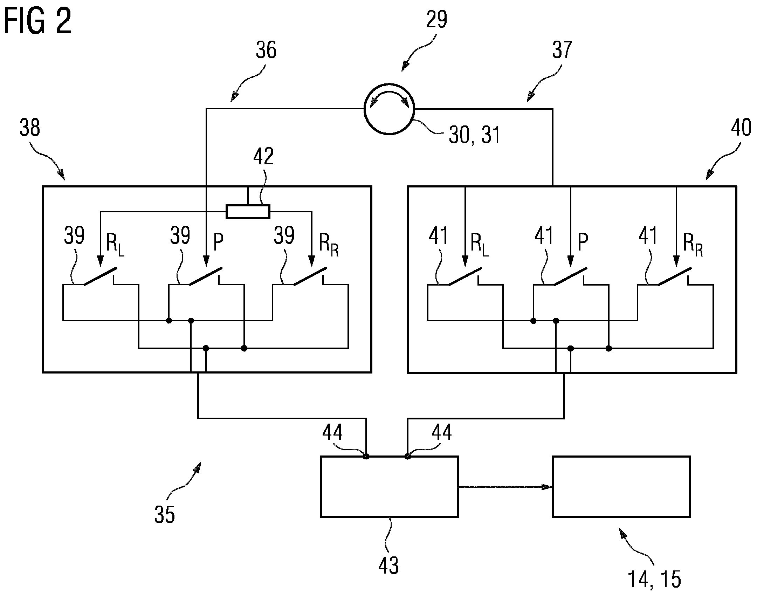

Zudem weist die Magnetresonanzvorrichtung 10 für eine Steuerung des Patiententischs 15, insbesondere für eine Steuerung einer Bewegung des Patiententischs 15, eine Patiententischsteuerung 35 auf (Fig. 2). Die Patiententischsteuerung 35 umfasst die weitere Eingabeeinheit 29, insbesondere der druckempfindliche Drehschalter 31, und zumindest zwei Steuerpfade 36, 37. Mittels der zwei Steuerpfade 36, 37 ist eine Benutzereingabe mittels der Benutzerschnittstelle 23, insbesondere der weiteren Eingabeeinheit 29 und/oder des druckempfindlichen Drehschalters 31 erfassbar. Dabei ist eine Erfassung einer Benutzereingabe an dem Bedienelement 30, insbesondere dem druckempfindlichen Drehschalter 31, mittels eines ersten Steuerpfads 36 der beiden Steuerpfade 36, 37 unabhängig von einer Erfassung der Benutzereingabe an dem Bedienelement 30, insbesondere dem druckempfindlichen Drehschalter 31, mittels des zweiten Steuerpfads 37 der beiden Steuerpfade 36, 37.

Für die unabhängige Erfassung der Benutzereingabe an dem Bedienelement 30, insbesondere dem druckempfindlichen Drehschalter 31, mittels der beiden Steuerpfade 36, 37 weist im vorliegenden Ausführungsbeispiel der erste Steuerpfad 36 eine Schalteinheit 38 auf (Fig. 2). Die Schalteinheit 38 des ersten Steuerpfads 36 umfasst eine Anzahl an Schaltelementen 39, wobei die Anzahl der Schaltelemente 39 abhängig ist von einer Anzahl an unterschiedlichen Einstellpositionen für das Bedienelement 30, insbesondere dem druckempfindlichen Drehschalter 31. Zudem weist auch der zweite Steuerpfad 37 eine Schalteinheit 40 auf. Die Schalteinheit 40 des zweiten Steuerpfads 37 umfasst eine Anzahl an Schaltelementen 41, wobei die Anzahl der Schaltelemente 41 abhängig ist von einer Anzahl an unterschiedlichen Einstellpositionen für das Bedienelement 30, insbesondere dem druckempfindlichen Drehschalter 31.

Aufgrund der Ausbildung des Bedienelements 30 als druckempfindlicher Drehschalter 31 weist das Bedienelement 30, insbesondere der druckempfindliche Drehschalter 31, im vorliegenden Ausführungsbeispiel drei Einstellpositionen auf. Eine erste Einstellposition des Bedienelements 30, insbesondere des druckempfindlichen Drehschalters 31, umfasst ein Rotieren und/oder Drehen R_{L} des druckempfindlichen Drehschalters 31 nach links. Eine zweite Einstellposition umfasst des Bedienelements 30, insbesondere des druckempfindlichen Drehschalters 31, ein Drücken P des druckempfindlichen Drehschalters 31. Eine dritte Einstellposition des Bedienelements 30, insbesondere des druckempfindlichen Drehschalters 31, umfasst ein Rotieren und/oder Drehen R_{R} des druckempfindlichen Drehschalters 31 nach rechts. Somit umfassen die Schalteinheit 38 des ersten Steuerpfads 36 und die Schalteinheit 40 des zweiten Steuerpfads 37 jeweils drei Schaltelemente 39, 41 (Fig. 2).

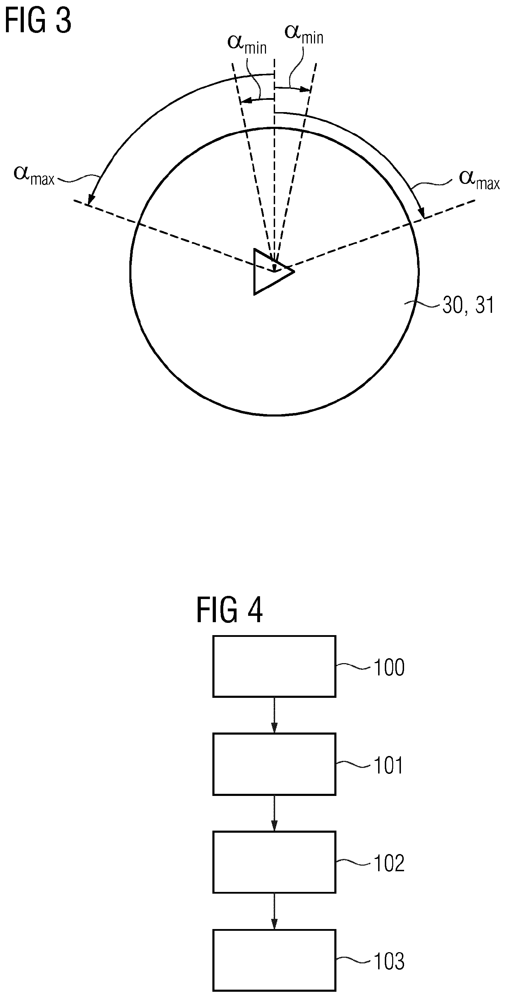

Des Weiteren ist die Patiententischsteuerung 35 dazu ausgelegt und/oder ausgebildet, eine Intensität einer Eingabe an dem Bedienelement 30, insbesondere an dem druckempfindlichen Drehschalter 31, zu erfassen. Hierzu weist die Patiententischsteuerung 35, insbesondere der erste Steuerpfad 36 der Patiententischsteuerung 35, eine Intensitätsmesseinheit 42 auf. Im vorliegenden Ausführungsbeispiel weist die Intensitätsmesseinheit 42 ein Potentiometer auf. Die Intensität der Eingabe an dem Bedienelement 30, insbesondere dem druckempfindlichen Drehschalter 31, weist insbesondere einen Grad einer Drehung an dem druckempfindlichen Drehschalter 31 auf. Mittels des Grads der Drehung an dem druckempfindlichen Drehschalter 31 kann von einem Benutzer, insbesondere dem medizinischen Bedienpersonal, zumindest eine Bewegungsparameter, insbesondere eine Geschwindigkeit, des Patiententischs 15 eingestellt werden (Fig. 3). Hierzu ist das Potentiometer derart ausgebildet, dass ein Rotieren und/oder Drehen, insbesondere ein Rotieren und/oder Drehen R_{L} nach links oder ein Rotieren und/oder Drehen R_{R} nach rechts, an dem druckempfindlichen Drehschalter 31 einen Mindestwinkel αₘᵢₙ übersteigen muss, um eine versehentliche minimale Drehung des druckempfindlichen Drehschalters 31 bei einer Benutzereingabe auszuschließen. Bei einem Weiterdrehen des druckempfindlichen Drehschalters 31 bis zu einem maximalen Drehwinkel αₘₐₓ kann eine Geschwindigkeit des Patiententischs 15 eingestellt werden (Fig. 4). Alternativ hierzu kann der zumindest eine einstellbare Bewegungsparameter auch eine Strecke, die der Patiententisch 15 zurücklegen soll, und/oder weitere, dem Fachmann als sinnvoll erscheinende Parameter umfassen (Fig. 2 und 3).

In einer alternativen Ausgestaltung der Erfindung kann der erste Steuerpfad 36 zur Erfassung einer Benutzereingabe an dem Bedienelement 29 nur eine Intensitätsmesseinheit 42, insbesondere das Potentiometer, umfassen und nur der zweite Steuerpfad 36 zur Erfassung einer Benutzereingabe an dem Bedienelement 29 eine Schalteinheit 40 mit Schaltelementen 41 umfasst. Zudem kann auch der zweite Steuerpfad 37 in einer alternativen Ausgestaltung der Erfindung eine Intensitätsmesseinheit 42, insbesondere ein Potentiometer, umfassen.

Die Patiententischsteuerung 35 weist zudem eine Recheneinheit 43 auf, wobei die Recheneinheit 43 zur Generierung eines Steuerbefehls für den Patiententisch 15, insbesondere für eine Bewegung des Patiententischs 15, ausgelegt ist. Die Recheneinheit 43 ist im vorliegenden Ausführungsbeispiel separat zur Systemsteuereinheit 22 ausgebildet und mittels einer Datenübertragungseinheit mit der Systemsteuereinheit 22 verbunden. In einer alternativen Ausgestaltung der Erfindung kann die Recheneinheit 43 von der Systemsteuereinheit 22 umfasst sein (Fig. 2).

Zur Generierung von Steuerbefehlen für eine Steuerung des Patiententischs 15, insbesondere für eine Steuerung einer Bewegung des Patiententischs 15, weist die Recheneinheit 43 eine erforderliche Steuersoftware und/oder Steuerprogramme auf, die in einer nicht näher dargestellten Speichereinheit der Recheneinheit 43 gespeichert sind. Bei einem Ausführen der Steuersoftware und/oder der Steuerprogramme durch einen nicht näher dargestellten Prozessor der Recheneinheit 43 erfolgt eine Steuerung des Patiententischs 15, insbesondere eine Steuerung einer Bewegung des Patiententischs 15. Hierzu werden mittels der Steuersoftware und/oder der Steuerprogramme Steuerparameter generiert und an den Patiententisch 15 geleitet, sofern die erforderliche Eingabedaten in der Recheneinheit 43 vorliegen.

Zur Übertragung von ersten Eingabedaten einer Eingabe an dem Bedienelement 30, insbesondere dem druckempfindlichen Drehschalter 31, mittels des ersten Steuerpfads 36 und der Übertragung von zweiten Eingabedaten einer Eingabe an dem Bedienelement 30, insbesondere dem druckempfindlichen Drehschalter 31, mittels des zweiten Steuerpfads 37 weist die Recheneinheit 43 zwei separat ausgebildete Eingänge 44, insbesondere zwei separat ausgebildet Dateneingänge, für die beiden Steuerpfade 36, 37 auf (Fig. 2). In der Recheneinheit 43 werden die ersten Eingabedaten und die zweiten Eingabedaten verarbeitet und, sofern die ersten Eingabedaten eine mit den zweiten Eingabedaten übereinstimmende Eingabeinformation aufweisen, ein Steuerbefehl zur Bewegung des Patiententischs 15 generiert. Liegt beispielsweise aus beiden Steuerpfaden 36, 37 eine Information für ein Einfahren des Patiententischs 15 in den Patientenaufnahmebereich 12 vor, wird von Recheneinheit 43 ein entsprechender Steuerbefehl generiert und an den Patiententisch 15 weitergegeben. Liegen dagegen widersprüchliche Informationen durch die beiden Steuerpfade 36, 37 hinsichtlich einer Bewegung des Patiententischs 15 in der Recheneinheit 43 vor, wird von der Recheneinheit 43 kein Steuerbefehl zur Bewegung des Patiententischs 15 generiert. Beispielsweise kann dabei nur ein Steuerpfad 36, 37 der beiden Steuerpfade 36, 37 eine Information für eine Bewegung des Patiententischs 15 umfassen, während durch den weiteren Steuerpfad 36, 37 der beiden Steuerpfade 36, 37 keine Informationen für eine Bewegung des Patiententischs 15 vorliegen. Dabei kann es auch sein, dass bei Vorliegen von widersprüchlichen Informationen durch die beiden Steuerpfade 36, 37 hinsichtlich einer Bewegung des Patiententischs 15 in der Recheneinheit 43 von der Recheneinheit 43 ein Stoppbefehl und/oder ein Arretierbefehl generiert wird, der eine Bewegung des Patiententischs 15 verhindert und/oder blockiert und/oder eine aktuelle Bewegung des Patiententischs 15 stoppt.

Die dargestellten Magnetresonanzvorrichtung 10 kann selbstverständlich weitere Komponenten umfassen, die Magnetresonanzvorrichtungen 10 gewöhnlich aufweisen. Eine allgemeine Funktionsweise einer Magnetresonanzvorrichtung 10 ist zudem dem Fachmann bekannt, so dass auf eine detaillierte Beschreibung der weiteren Komponenten verzichtet wird.

In Fig. 4 ist ein erfindungsgemäßes Verfahren zu einem Steuern einer Bewegung des Patiententischs 15 der Magnetresonanzvorrichtung 10 dargestellt. Das Verfahren wird automatisch und/oder selbsttätig von der Recheneinheit 43 ausgeführt und/oder gesteuert. Dementsprechend sind auch die in der Recheneinheit 43 gespeicherte Steuersoftware und/oder die Steuerprogramme zu einer automatischen und/oder selbsttätigen Ausführung einer Steuerung einer Bewegung des Patiententischs 15 der Magnetresonanzvorrichtung 10 ausgebildet und/oder ausgelegt.

In einem ersten Verfahrensschritt 100 erfolgt ein Erfassen von Eingabedaten einer Eingabe an dem Bedienelement 30, insbesondere an dem druckempfindlichen Drehschalter 31. Hierbei werden in dem ersten Verfahrensschritt 100 erste Eingabedaten an dem Bedienelement 30, insbesondere an dem druckempfindlichen Drehschalter 31, mittels des ersten Steuerpfads 36 und zweite Eingabedaten an dem Bedienelement 30, insbesondere an dem druckempfindlichen Drehschalter 31, mittels des zweiten Steuerpfads 37 erfasst. Dabei werden die ersten Eingabedaten mittels des ersten Steuerpfads 36 unabhängig von den zweiten Eingabedaten des zweiten Steuerpfads 37 erfasst. Die Eingabe an dem Bedienelement 30, insbesondere an dem druckempfindlichen Drehschalter 31, umfasst eine Drehbewegung und/oder eine Drückbewegung des Bedienelements 30, insbesondere des druckempfindlichen Drehschalters 31.

In einem an den ersten Verfahrensschritt 100 anschließenden zweiten Verfahrensschritt 101 erfolgt ein Übertragen der ersten Eingabedaten an die Recheneinheit 43 mittels des ersten Steuerpfads 36 und eine Übertragen der zweiten Eingabedaten an die Recheneinheit 43 mittels des zweiten Steuerpfads 37. Die Übertragung der ersten Eingabedaten mittels des ersten Steuerpfads 36 ist dabei unabhängig von einer Übertragung der zweiten Eingabedaten mittels des zweiten Steuerpfads 37.

In einem dritten Verfahrensschritt 102 erfolgt anschließend ein Auswerten der ersten Eingabedaten und der zweiten Eingabedaten mittels der Recheneinheit 43. Dabei wird von der Recheneinheit 43 ein Steuerbefehl zur Steuerung des Patiententischs 15 generiert, sobald die ersten Eingabedaten die zweiten Eingabedaten eine übereinstimmende Eingabeinformation umfassen. Liegt beispielsweise aus beiden Steuerpfaden 36, 37 eine Information für ein Einfahren des Patiententischs 15 in den Patientenaufnahmebereich 15 vor, wird von Recheneinheit 43 ein entsprechender Steuerbefehl generiert und an den Patiententisch 15 weitergegeben. Liegen dagegen widersprüchliche Informationen durch die beiden Steuerpfade 36, 37 hinsichtlich einer Bewegung des Patiententischs 15 in der Recheneinheit 43 vor, wird von der Recheneinheit 43 kein Steuerbefehl zur Bewegung des Patiententischs 15 generiert. Beispielsweise kann dabei nur ein Steuerpfad 36, 37 der beiden Steuerpfade 36, 37 eine Information für eine Bewegung des Patiententischs 15 umfassen, während bei einem weiteren Steuerpfad 36, 37 der beiden Steuerpfade 36, 37 keine Informationen für eine Bewegung des Patiententischs 15 vorliegen. Dabei kann es auch sein, dass bei Vorliegen von widersprüchlichen Informationen durch die beiden Steuerpfade 36, 37 hinsichtlich einer Bewegung des Patiententischs 15 in der Recheneinheit 43 von der Recheneinheit 43 ein Stoppbefehl und/oder ein Arretierbefehl generiert wird, der eine Bewegung des Patiententischs 15 verhindert und/oder blockiert und/oder eine aktuelle Bewegung des Patiententischs 15 stoppt.

Bevorzugt liegt in diesem dritten Verfahrensschritt 102 in der Recheneinheit 43 auch eine aktuelle Position des Patiententischs 15 und/oder ein aktueller Bewegungszustand des Patiententischs 15 vor, wobei der Steuerbefehl in Abhängigkeit von der aktuellen Position des Patiententischs 15 und/oder dem aktuellen Bewegungszustand des Patiententischs 15 von der Recheneinheit 43 generiert wird. Die aktuelle Position und/oder ein aktueller Bewegungszustand des Patiententischs 15 kann dabei in der Speichereinheit der Recheneinheit 43 gespeichert sein. Zudem kann die Magnetresonanzvorrichtung 10 eine Erfassungseinheit zur Erfassung einer aktuellen Position und/oder eines aktuellen Bewegungszustands des Patiententischs 15 aufweisen, wie beispielsweise eines Sensoreinheit.

Des Weiteren kann in der Recheneinheit 43 auch eine Zielposition des Patiententischs 15 vorgegeben sein, so dass die Recheneinheit 43 eine Bewegung des Patiententischs 15 zusätzlich in Abhängigkeit von der Zielposition des Patiententischs 15 einstellt. Die Zielposition des Patiententischs 15 kann beispielsweise eine Isozentrums-Position des Patiententischs 15 umfassen, in der sich der zu untersuchende Bereich des Patienten 13 im Isozentrum des Patientenaufnahmebereichs 12 befindet. Die Isozentrums-Position des Patiententischs 15 als Zielposition kann beispielsweise vor einer Magnetresonanzmessung vorliegen. Zudem kann die Zielposition des Patiententischs 15 eine Home-Position des Patiententischs 15 umfassen. Die Home-Position des Patiententischs 15 umfasst diejenige Position des Patiententischs 15, in der sich der Patiententisch 15 außerhalb des Patientenaufnahmebereichs 12 befindet, jedoch bereit zum Einfahren in den Patientenaufnahmebereich 12. Die Home-Position des Patiententischs 15 als Zielposition kann beispielsweise nach einer Magnetresonanzmessung vorliegen, wenn der Patient 13 wieder aus dem Patientenaufnahmebereich 12 herausgefahren werden soll.

Zudem kann der von der Recheneinheit 43 generierte Steuerbefehl eine Richtung einer Bewegung des Patiententischs 15 umfassen, wobei die Richtung der Bewegung des Patiententischs 15 abhängig sein kann von einer Drehrichtung des Bedienelements 30, insbesondere des druckempfindlichen Drehschalters 31. Im vorliegenden Ausführungsbeispiel bewirkt ein Rotieren und/oder Drehen R_{R} des Bedienelements 30, insbesondere des druckempfindlichen Drehschalters 31, nach rechts eine Vorwärtsbewegung des Patiententischs 15 und/oder es kann durch ein Rotieren und/oder Drehen R_{R} nach rechts des Bedienelements 30, insbesondere des druckempfindlichen Drehschalters 31, eine Geschwindigkeit einer Vorwärtsbewegung des Patiententischs 15 eingestellt werden. Des Weiteren bewirkt ein Rotieren und/oder Drehen R_{L} des Bedienelements 30, insbesondere des druckempfindlichen Drehschalters 31, nach links eine Rückwärtsbewegung des Patiententischs 15 und/oder es kann durch ein Rotieren und/oder Drehen R_{L} nach links des Bedienelements 30, insbesondere des druckempfindlichen Drehschalters 31, eine Geschwindigkeit einer Rückwärtsbewegung des Patiententischs 15 eingestellt werden.

Daran anschließend erfolgt in einem vierten Verfahrensschritt 103 ein Ausführen des Steuerbefehls zur Steuerung der Bewegung des Patiententischs 15. Hierzu wird der Steuerbefehl von der Recheneinheit 43 an den Patiententisch 15, insbesondere eine nicht näher dargestellte Antriebseinheit des Patiententischs 15, übertragen.

Eine Ausgangsposition eines ersten Beispiels für eine kontextabhängige Generierung eines Steuerbefehls zur Steuerung einer Bewegung des Patiententischs 15 kann sein, dass der Patiententisch 15 sich in einer abgesenkten Position befindet, beispielsweise für eine Patientenvorbereitung. Zudem kann als Zielposition des Patiententischs 15 die Isozentrums-Position vorgegeben sein, wie dies zu Beginn einer Untersuchung der Fall sein kann. Durch Drücken P des Bedienelements 30, insbesondere des druckempfindlichen Drehschalters 31, und einem anschließenden Drehen R_{R} des Bedienelements 30, insbesondere des druckempfindlichen Drehschalters 31, nach rechts wird zunächst ein Steuerbefehl generiert, der den Patiententisch 15 von der abgesenkten Position des Patiententischs 15 in die Home-Position des Patiententischs 15 anhebt. In der Home-Position des Patiententischs 15 befindet sich der Patiententisch außerhalb des Patientenaufnahmebereichs 12, jedoch bereit für ein Einfahren des Patiententischs 15 in den Patientenaufnahmebereich 12. Zudem wird ein Steuerbefehl generiert, der eine Horizontalbewegung des Patiententischs 15, insbesondere ein Einfahren des Patiententischs 15, in den Patientenaufnahmebereich 12 derart bewirkt, dass der Patiententisch 15 in die Zielposition des Patiententischs 15, insbesondere in die Isozentrums-Position des Patiententischs 15, fährt. In dem vierten Verfahrensschritt 103 wird zunächst der Patiententisch 15 in die Home-Position des Patiententischs 15 angehoben und anschließend in den Patientenaufnahmebereich 12 hineinbewegt bis ein zu untersuchender Bereich des Patienten 13 im Isozentrum angeordnet ist bzw. der Patiententisch 15 in der Isozentrums-Position des Patiententischs 15 angeordnet ist.

Befindet sich beispielsweise der Patiententisch 15 bereits in der Home-Position des Patiententischs 15 und ist als Zielposition des Patiententischs 15 die Isozentrums-Position des Patiententischs 15 vorgegeben, bewirkt ein Drücken P des Bedienelements 30, insbesondere des druckempfindlichen Drehschalters 31, dass der Patiententisch 15 automatisch eine Horizontalbewegung ausführt, bis der zu untersuchende Bereich des Patienten 13 im Isozentrum des Patientenaufnahmebereichs 12 angeordnet ist bzw. der Patiententisch 15 in der Isozentrums-Position des Patiententischs 15 angeordnet ist. Aufgrund der Drückbewegung an dem Bedienelement 30, insbesondere an dem druckempfindlichen Drehschalter 31, wird von der Recheneinheit 43 ein entsprechender Steuerbefehl generiert, der eine entsprechende Horizontalbewegung des Patiententischs 15 bei einem Ausführen des Steuerbefehls bewirkt. Dabei kann die Horizontalbewegung des Patiententischs 15 in die Isozentrums-Position des Patiententischs 15 mit maximaler Geschwindigkeit erfolgen. Wird während der Bewegung des Patiententischs 15 vom medizinischen Bedienpersonal eine Gefahr eine Kollision des Patiententischs 15 und/oder des Patienten 13 und/oder eine Zubehöreinheit mit der Scannereinheit 11 erkannt, kann durch ein Drehen R_{R}, des Bedienelements 30, insbesondere des druckempfindlichen Drehschalters 31, beispielsweise durch ein Drehen R_{R} nach rechts des druckempfindlichen Drehschalters 31, eine Geschwindigkeit, insbesondere eine langsamere Geschwindigkeit, des Patiententischs 15 eingestellt werden. Zudem kann vom medizinischen Bedienpersonal hierbei die Geschwindigkeit des Patiententischs 15 wieder auf ein Maximum angehoben werden, sobald die kritische Situation vorbei ist. Dabei muss das medizinische Bedienpersonal erneut das Bedienelement 30, insbesondere den druckempfindlichen Drehschalter 31, betätigen, insbesondere eine maximale Drehbewegung nach rechts ausführen, und von der Recheneinheit 43 wird eine höhere und/oder maximale Geschwindigkeit des Patiententischs 15 eingestellt.

Befindet sich beispielsweise der Patiententisch 15 in einer Position zwischen der Home-Position des Patiententischs 15 und der Isozentrums-Position des Patiententischs 15 und ist als Zielposition des Patiententischs 15 die Isozentrums-Position des Patiententischs 15 vorgegeben, bewirkt ein Drücken P des Bedienelements 30, insbesondere des druckempfindlichen Drehschalters 31, dass der Patiententisch 15 automatisch eine Horizontalbewegung ausführt, bis der zu untersuchende Bereich des Patienten 13 im Isozentrum des Patientenaufnahmebereichs 12 angeordnet ist bzw. der Patiententisch 15 in der Isozentrums-Position des Patiententischs 15 angeordnet ist. Aufgrund der Drückbewegung an dem Bedienelement 30, insbesondere an dem druckempfindlichen Drehschalter 31, wird von der Recheneinheit 43 ein entsprechender Steuerbefehl generiert, der eine entsprechende Horizontalbewegung des Patiententischs 15 bei einem Ausführen des Steuerbefehls bewirkt. Dabei kann die Horizontalbewegung des Patiententischs 15 in die Isozentrums-Position des Patiententischs 15 mit maximaler Geschwindigkeit erfolgen. Wird während der Bewegung des Patiententischs 15 vom medizinischen Bedienpersonal eine Gefahr eine Kollision des Patiententischs 15 und/oder des Patienten 13 und/oder eine Zubehöreinheit mit der Scannereinheit 11 erkannt, kann durch ein Drehen R_{R}, des Bedienelements 30, insbesondere des druckempfindlichen Drehschalters 31, beispielsweise durch ein Drehen R_{R} nach rechts des druckempfindlichen Drehschalters 31, eine Geschwindigkeit, insbesondere eine langsamere Geschwindigkeit, des Patiententischs 15 eingestellt werden. Zudem kann vom medizinischen Bedienpersonal hierbei die Geschwindigkeit des Patiententischs 15 wieder auf ein Maximum angehoben werden, sobald die kritische Situation vorbei ist. Dabei muss das medizinische Bedienpersonal erneut das Bedienelement 30, insbesondere den druckempfindlichen Drehschalter 31, betätigen, insbesondere eine maximale Drehbewegung nach rechts ausführen, und von der Recheneinheit 43 wird eine höhere und/oder maximale Geschwindigkeit des Patiententischs 15 eingestellt.

Befindet sich beispielsweise der Patiententisch 15 in einer Position zwischen der Home-Position des Patiententischs 15 und der Isozentrums-Position des Patiententischs 15 und ist als Zielposition des Patiententischs 15 die Home-Position des Patiententischs 15 vorgegeben, bewirkt ein Drücken P des Bedienelements 30, insbesondere des druckempfindlichen Drehschalters 31, dass der Patiententisch 15 automatisch eine Horizontalbewegung ausführt, bis der Patiententisch 15 in der Home-Position ist. Aufgrund der Drückbewegung an dem Bedienelement 30, insbesondere an dem druckempfindlichen Drehschalter 31, wird von der Recheneinheit 43 ein entsprechender Steuerbefehl generiert, der eine entsprechende Horizontalbewegung des Patiententischs 15 bei einem Ausführen des Steuerbefehls bewirkt. Dabei kann die Horizontalbewegung des Patiententischs 15 in die Home-Position des Patiententischs 15 mit maximaler Geschwindigkeit erfolgen. Wird während der Bewegung des Patiententischs 15 vom medizinischen Bedienpersonal eine Gefahr eine Kollision des Patiententischs 15 und/oder des Patienten 13 und/oder eine Zubehöreinheit mit der Scannereinheit 11 erkannt, kann durch ein Drehen R_{L}, des Bedienelements 30, insbesondere des druckempfindlichen Drehschalters 31, beispielsweise durch ein Drehen R_{L} nach links des druckempfindlichen Drehschalters 31, eine Geschwindigkeit, insbesondere eine langsamere Geschwindigkeit, des Patiententischs 15 eingestellt werden. Zudem kann vom medizinischen Bedienpersonal hierbei die Geschwindigkeit des Patiententischs 15 wieder auf ein Maximum angehoben werden, sobald die kritische Situation vorbei ist. Dabei muss das medizinische Bedienpersonal erneut das Bedienelement 30, insbesondere den druckempfindlichen Drehschalter 31, betätigen, insbesondere eine maximale Drehbewegung nach links ausführen, und von der Recheneinheit 43 wird eine höhere und/oder maximale Geschwindigkeit des Patiententischs 15 eingestellt.

Befindet sich beispielsweise der Patiententisch 15 in der Home-Position des Patiententischs 15 und der Patiententisch 15 soll abgesenkt werden, wie dies insbesondere nach einer Magnetresonanzuntersuchung sinnvoll ist, wird durch Drehen R_{L} des Bedienelements 30, insbesondere des druckempfindlichen Drehschalters 31, nach links eine Absenkbewegung initiiert. Aufgrund der Drehbewegung an dem Bedienelement 30, insbesondere an dem druckempfindlichen Drehschalter 31, wird von der Recheneinheit 43 ein entsprechender Steuerbefehl generiert, der eine entsprechende Absenkbewegung des Patiententischs 15 bei einem Ausführen des Steuerbefehls bewirkt. Die Absenkbewegung kann dabei solange ausgeführt werden, sie das Bedienelement 30, insbesondere der druckempfindliche Drehschalter 31, von dem Benutzer, insbesondere dem medizinisches Bedienpersonal, nach links gedreht bleibt oder eine Endposition beim Absenken des Patiententischs 15 erreicht ist.

Mittels der weiteren Ausgabeeinheit 33, insbesondere des Displays 34, kann hierbei dem Benutzer, insbesondere dem medizinischen Bedienpersonal, stets eine aktuelle Position des Patiententischs 15 bezüglich der Home-Position des Patiententischs 15 in vertikaler Richtung und/oder bezüglich der Home-Position und/oder der Isozentrums-Position es Patiententischs 15 in horizontaler Richtung angezeigt werden.

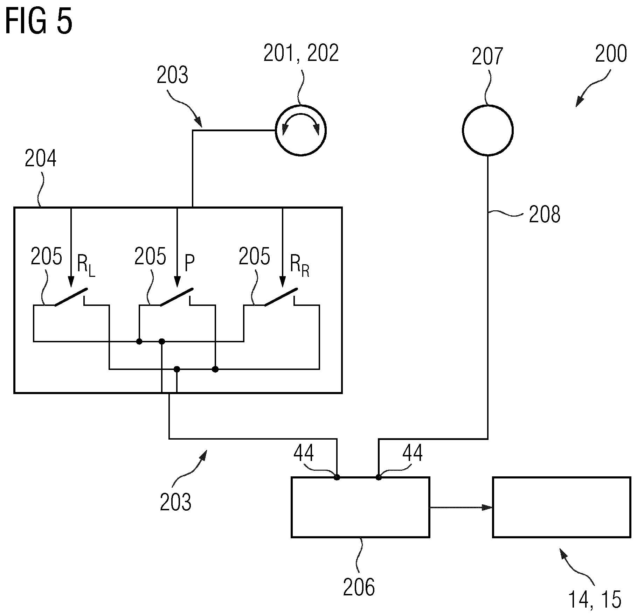

In Fig. 5 ist ein alternatives Ausführungsbeispiel einer Patiententischsteuerung 200 zur Steuerung eines Patiententischs 15 einer Magnetresonanzvorrichtung 10 dargestellt, das nicht unter den Schutzumfang der Erfindung fällt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in den Fig. 1 bis 3, wobei bezüglich gleichbleibender Bauteile, Merkmale und Funktionen auf die Beschreibung des Ausführungsbeispiels in den Fig. 1 bis 3 verwiesen wird.

Die Patiententischsteuerung 200 in Fig. 5 weist ein Bedienelement 201 auf, das einen druckempfindlichen Drehschalter 202 aufweist und das gemäß den Ausführungen zu den Fig. 1 bis 3 ausgebildet ist. Für eine Übertragung der mittels des Bedienelements 201 erfassten ersten Eingabedaten weist die Patiententischsteuerung 200 einen ersten Steuerpfad 203 auf, der eine Schalteinheit 204 mit drei Schaltelementen 205 aufweist. Zudem weist die Patiententischsteuerung 200 eine Recheneinheit 206 auf, die gemäß den Ausführungen zu den Fig. 1 bis 3 ausgebildet ist.

Des Weiteren weist die Patiententischsteuerung 200 ein weiteres Bedienelement 207 auf, das mit einem zweiten Steuerpfad 208 gekoppelt ist. Das zweite Bedienelement 207 kann dabei eine Taste und/oder einen Schalter und/oder weitere, dem Fachmann als sinnvoll erscheinende Bedienelemente 207 und/oder Eingabeelement umfassen. Das zweite Bedienelement 207 weist eine Arretierfunktion des Patiententischs 15 auf. Die mittels des zweiten Bedienelements 207 erfassten zweiten Eingabedaten, die einen Aktivierungszustand der Arretierfunktion des Patiententischs 15 umfassen, werden mittels des zweiten Steuerpfads 208 an die Recheneinheit 43 übertragen. Durch ein Drücken des zweiten Bedienelements 207 kann dabei die Arretierfunktion des Patiententischs 15 aktiviert werden. Die Arretierfunktion umfasst eine elektronische Arretierfunktion des Patiententischs 15, so dass kein Steuerbefehl für eine Bewegung des Patiententischs 15 zum Patiententisch 15 gelangen kann und/oder kein Steuerbefehl für eine Bewegung des Patiententischs 15 in der Recheneinheit 206 generiert wird.

Solange das zweite Bedienelement 207 gedrückt ist, bleibt die Arretierfunktion für den Patiententisch 15 aktiviert. Erst wenn das zweite Bedienelement 207 nicht mehr gedrückt ist und damit die Arretierung für den Patiententisch 15 aufgehoben ist, kann bei einer entsprechenden Eingabe an dem ersten Bedienelement 201 eine Steuerung einer Bewegung des Patiententischs 15 durch die Recheneinheit 206 erfolgen. Dabei werden in der Recheneinheit 206 die ersten Eingabedaten des ersten Steuerpfads 203 und die zweiten Eingabedaten des zweiten Steuerpfads 208 verarbeitet und, sofern die ersten Eingabedaten eine mit den zweiten Eingabedaten übereinstimmende Eingabeinformation aufweisen, ein Steuerbefehl zur Bewegung des Patiententischs 15 generiert. Liegt beispielsweise aus dem ersten Steuerpfad 203 eine Information für ein Einfahren des Patiententischs 15 in den Patientenaufnahmebereich 12 vor und aus dem zweiten Steuerpfad 208 eine Freigabe für eine Bewegung des Patiententischs 15 vor, wird von Recheneinheit 206 ein entsprechender Steuerbefehl zur Bewegung des Patiententischs 15 generiert und an den Patiententisch 15 weitergegeben. Liegen dagegen widersprüchliche und/oder gegensätzlich Informationen durch die beiden Steuerpfade 203, 208 hinsichtlich einer Bewegung des Patiententischs 15 in der Recheneinheit 206 vor, indem beispielweise die Arretierfunktion aktiviert ist, wird von der Recheneinheit 206 kein Steuerbefehl zur Bewegung des Patiententischs 15 generiert.

Obwohl die Erfindung im Detail durch die bevorzugte Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen, welcher durch die beigefügten Ansprüche definiert ist.

## Patentansprüche

1. Magnetresonanzvorrichtung mit einer Scannereinheit (11), einem bewegbaren Patiententisch (15), einer Patiententischsteuerung (35) und einer Benutzerschnittstelle für eine Benutzereingabe zur Einstellung einer Bewegung des Patiententischs, wobei die Benutzerschnittstelle ein Bedienelement (29) aufweist, das einen Drehschalter (31) umfasst, wobei die Patiententischsteuerung zumindest zwei Steuerpfade (36,37) und eine Recheneinheit (43) umfasst,
wobei ein erster der zumindest zwei Steuerpfade dazu ausgebildet ist, erste Eingabedaten der Benutzereingabe am Drehschalter zu erfassen und ein zweiter der zumindest zwei Steuerpfade dazu ausgebildet ist, zweite Eingabedaten der Benutzereingabe am Drehschalter zu erfassen,
wobei die Recheneinheit zur Generierung eines Steuerbefehls für den Patiententisch ausgelegt ist und die Recheneinheit zwei separate Eingänge aufweist und jeder der beiden Eingänge einem der beiden Steuerpfade zugeordnet ist,
wobei die Recheneinheit (43) dazu ausgelegt ist, die ersten Eingabedaten und die zweiten Eingabedaten auszuwerten und den Steuerbefehls zur Steuerung des Patiententischs (15) nur dann zu generieren, wenn die ersten Eingabedaten und die zweiten Eingabedaten eine übereinstimmende Eingabeinformation umfassen, wobei die Patiententischsteuerung dazu ausgebildet ist, in Abhängigkeit eines Umfangs einer Drehbewegung des Drehschalters eine Geschwindigkeit für eine Einfahrbewegung und/oder eine Ausfahrbewegung des Patiententischs zu bestimmen.

2. Magnetresonanzvorrichtung nach Anspruch 1,
**dadurch gekennzeichnet, dass** ein erster Steuerpfad der beiden Steuerpfade eine Steuerelektronik aufweist, die unabhängig und/oder separat zu einer Steuerelektronik des zweiten Steuerpfads ausgebildet ist.

3. Magnetresonanzvorrichtung nach einem der vorhergehenden Ansprüche,
**dadurch gekennzeichnet, dass** zumindest ein Steuerpfad der beiden Steuerpfade eine Schalteinheit mit einer Anzahl an Schaltelementen umfasst, wobei die Anzahl der Schaltelemente abhängig ist von einer Anzahl an unterschiedlichen Einstellpositionen des Drehschalters der Benutzerschnittstelle.

4. Magnetresonanzvorrichtung nach Anspruch 3,
**dadurch gekennzeichnet, dass** die Schalteinheit drei Schaltelemente aufweist.

5. Magnetresonanzvorrichtung nach einem der vorhergehenden Ansprüche,
**dadurch gekennzeichnet, dass** zumindest ein Steuerpfad der beiden Steuerpfade eine Intensitätsmesseinheit zur Erfassung einer Intensität einer Eingabe des Drehschalters aufweist.

6. Magnetresonanzvorrichtung nach einem der vorhergehenden Ansprüche,
**dadurch gekennzeichnet, dass** mittels der Patiententischsteuerung zumindest ein Bewegungsparameter zur Steuerung des Patiententischs in Abhängigkeit der erfassten Intensität der mittels des Drehschalters getätigten Eingabe einstellbar ist.

7. Verfahren zu einem Steuern einer Bewegung eines Patiententischs einer Magnetresonanzvorrichtung, die nach einem der vorhergehenden Ansprüche ausgebildet ist, wobei das Verfahren die folgenden Verfahrensschritte umfasst:
- Erfassen von ersten Eingabedaten einer Eingabe an einem Bedienelement mittels eines ersten Steuerpfads,
- Erfassen von zweiten Eingabedaten der Eingabe an dem Bedienelement mittels eines zweiten Steuerpfads,
- Übertragen der ersten Eingabedaten und der zweiten Eingabedaten an eine Recheneinheit,
- Auswerten der ersten Eingabedaten und der zweiten Eingabedaten mittels der Recheneinheit und Generieren eines Steuerbefehls zur Steuerung des Patiententischs, wobei das Generieren des Steuerbefehls mittels der Recheneinheit erfolgt, sobald die ersten Eingabedaten und die zweiten Eingabedaten eine übereinstimmente Eingabeinformation umfassen, und
- Ausführen des Steuerbefehls zur Steuerung der Bewegung des Patiententischs.

8. Verfahren nach Anspruch 7,
**dadurch gekennzeichnet, dass** das Erfassen der ersten Eingabedaten mittels des ersten Steuerpfads unabhängig von dem Erfassen der zweiten Eingabedaten mittels des zweiten Steuerpfads ist.

9. Verfahren nach einem der Ansprüche 7 bis 8,
**dadurch gekennzeichnet, dass** die Eingabe an dem zumindest einen Bedienelement eine Drehbewegung und/oder eine Drückbewegung an einem druckempfindlichen Drehschalter umfasst.

10. Verfahren nach einem der Ansprüche 7 bis 9,
**dadurch gekennzeichnet, dass** in der Recheneinheit zumindest ein aktueller Zustandsparameter des Patiententischs vorliegt und der Steuerbefehl in Abhängigkeit von dem zumindest einen aktuellen Zustandsparameter des Patiententischs von der Recheneinheit generiert wird.

11. Verfahren nach einem der Ansprüche 7 bis 10,
**dadurch gekennzeichnet, dass** der generierte Steuerbefehl eine Richtung einer Bewegung des Patiententischs umfasst, wobei die Richtung der Bewegung des Patiententischs abhängig von einer Drehrichtung des Bedienelements ist.

12. Computerprogrammprodukt, welches ein Programm umfasst und direkt in einen Speicher einer programmierbaren Recheneinheit einer Patiententischsteuerung einer Magnetresonanzvorrichtung nach Anspruch 1 ladbar ist, mit Programmmitteln, um ein Verfahren zu einem Steuern einer Bewegung eines Patiententischs einer Magnetresonanzvorrichtung nach einem der Ansprüche 7 bis 11 auszuführen, wenn das Programm in der Recheneinheit ausgeführt wird.

## Claims

1. Magnetic resonance device with a scanner unit (11), a moveable patient couch (15), a patient couch controller (35) and a user interface for a user input for adjusting a movement of the patient couch, wherein the user interface has a control element (29) which comprises a rotary switch (31), wherein the patient couch controller comprises at least two control paths (36, 37) and a computing unit (43), wherein a first of the at least two control paths is embodied to detect first input data of the user input on the rotary switch and a second of the at least two control paths is embodied to detect second input data of the user input on the rotary switch,
wherein the computing unit is designed to generate a control command for the patient couch and the computing unit has two separate inputs and each of the two inputs is assigned to one of the two control paths,
wherein the computing unit (43) is designed to evaluate the first input data and the second input data and only then to generate the control command for controlling the patient couch (15) when the first input data and the second input data comprise a concordant item of input information, wherein the patient couch controller is embodied to determine a speed for an insertion and/or retraction movement of the patient couch as a function of an extent of a rotational movement of the rotary switch.

2. Magnetic resonance device according to claim 1, **characterised in that** a first control path of the two control paths has a control electronics system, which is embodied independently of and/or separately from a control electronics system of the second control path.

3. Magnetic resonance device according to one of the preceding claims,
**characterised in that**
at least one control path of the two control paths comprises a switching unit with a number of switching elements, wherein the number of switching elements is dependent on a number of different adjustment positions of the rotary switch of the user interface.

4. Magnetic resonance device according to claim 3,
**characterised in that** the switching unit has three switching elements.

5. Magnetic resonance device according to one of the preceding claims,
**characterised in that** at least one control path of the two control paths has an intensity measuring unit for detecting an intensity of an input of the rotary switch.

6. Magnetic resonance device according to one of the preceding claims,
**characterised in that** at least one movement parameter for controlling the patient couch can be adjusted by means of the patient couch controller as a function of the detected intensity of the input executed by means of the rotary switch.

7. Method for controlling a movement of a patient couch of a magnetic resonance device, which is embodied according to one of the preceding claims, wherein the method comprises the following method steps:
- detecting first input data of an input on a control element by means of a first control path,
- detecting second input data of the input on the control element by means of a second control path,
- transmitting the first input data and the second input data to a computing unit,
- evaluating the first input data and the second input data by means of the computing unit and generating a control command for controlling the patient couch, wherein the control command is generated by means of the computing unit once the first input data and the second input data comprise a concordant item of input information, and
- executing the control command in order to control the movement of the patient couch.

8. Method according to claim 7,
**characterised in that** the detection of the first input data by means of the first control path is independent of the detection of the second input data by means of the second control path.

9. Method according to one of claims 7 to 8,
**characterised in that** the input on the at least one control element comprises a rotational movement and/or a push-through movement on a pressure-sensitive rotary switch.

10. Method according to one of claims 7 to 9,
**characterised in that** at least one current state parameter of the patient couch is present in the computing unit and the control command is generated by the computing unit as a function of the at least one current state parameter of the patient couch.

11. Method according to one of claims 7 to 10,
**characterised in that** the generated control command comprises a direction of a movement of the patient couch, wherein the direction of the movement of the patient couch is dependent on a rotational direction of the control element.

12. Computer program product, which comprises a program and can be loaded directly into a memory of a programmable computing unit of a patient couch controller of a magnetic resonance device according to claim 1, having program means in order to execute a method for controlling a movement of a patient couch of a magnetic resonance apparatus according to one of claims 7 to 11, when the program is executed in the computing unit.

## Revendications

1. Installation de résonance magnétique comprenant une unité (11) de scanner, une table (15) de patient mobile, une commande (35) de table de patient et une interface d'utilisateur pour une entrée d'utilisateur pour le réglage d'un mouvement de la table de patient, dans laquelle l'interface d'utilisateur a un élément (29) de commande, qui comprend un interrupteur (31) tournant, dans laquelle la commande de la table de patient comprend au moins deux chemins (36, 37) de commande et une unité (43) informatique,
dans laquelle un premier des au moins deux chemins de commande est constitué pour détecter des premières données de l'entrée d'utilisateur à l'interrupteur tournant et un deuxième des au moins deux chemins de commande est constitué pour détecter des deuxièmes données de l'entrée d'utilisateur à l'interrupteur tournant,
dans laquelle l'unité informatique est conçue pour la création d'une instruction de commande de la table de patient et l'unité informatique a deux entrées distinctes et à chacune des deux entrées est affecté l'un des deux chemins de commande,
dans laquelle l'unité (43) informatique est conçue pour évaluer les premières données d'entrée et les deuxièmes données d'entrée et pour ne créer l'instruction de commande pour la commande de la table (15) de patient que si les premières données d'entrée et les deuxièmes données d'entrée comprennent une information d'entrée en coïncidence,
dans laquelle la commande de la table de patient est constituée pour déterminer, en fonction d'un montant du mouvement de rotation de l'interrupteur tournant, une vitesse d'un mouvement d'entrée et/ou d'un mouvement de sortie de la table de patient.

2. Installation de résonance magnétique suivant la revendication 1,
**caractérisée en ce qu'**un premier chemin de commande des deux chemins de commande a une électronique de commande, qui est constituée de manière indépendante et/ou distincte d'une électronique de commande du deuxième chemin de commande.

3. Installation de résonance magnétique suivant l'une des revendications précédentes,
**caractérisée en ce qu'**au moins l'un des deux chemins de commande comprend une unité de coupure, ayant un nombre d'éléments de coupure, dont le nombre des éléments de coupure dépend du nombre de positions de réglage différentes de l'interrupteur tournant de l'interface d'utilisateur.

4. Installation de résonance magnétique suivant la revendication 3,
**caractérisée en ce que** l'unité de coupure a trois éléments de coupure.

5. Installation de résonance magnétique suivant l'une des revendications précédentes,
**caractérisée en ce qu'**au moins un chemin de commande des deux des chemins de commande a une unité de mesure de l'intensité pour la détection d'une intensité d'une entrée de l'interrupteur tournant.

6. Installation de résonance magnétique suivant l'une des revendications précédentes,
**caractérisée en ce que**, au moyen de la commande de la table de patient, au moins un paramètre de déplacement pour la commande de la table de patient peut être réglé en fonction de l'intensité détectée de l'entrée actionnée au moyen de l'interrupteur tournant.

7. Procédé de commande d'un mouvement d'une table de patient d'une installation de résonance magnétique, qui est constituée suivant l'une des revendications précédentes, dans lequel le procédé comprend les stades de procédé suivant :
- détection de premières données d'une entrée sur un élément de commande au moyen d'un premier chemin de commande,
- détection de deuxièmes données de l'entrée sur l'élément de commande au moyen d'un deuxième chemin de commande,
- transmission des premières données d'entrée et des deuxièmes données d'entrée à une unité informatique,
- évaluation des premières données d'entrée et des deuxièmes données d'entrée au moyen de l'unité informatique et création d'une instruction de commande pour la commande de la table de patient, dans lequel la création de l'instruction de commande s'effectue au moyen de l'unité informatique, dès que les premières données d'entrée et les deuxièmes données d'entrée comprennent une information d'entrée en coïncidence, et
- exécution de l'instruction de commande pour la commande du mouvement de la table de patient.

8. Procédé suivant la revendication 7,
**caractérisé en ce que** la détection des premières données d'entrée au moyen du premier chemin de commande est indépendante de la détection des deuxièmes données d'entrée au moyen du deuxième chemin de commande.

9. Procédé suivant l'une des revendications 7 à 8,
**caractérisé en ce que** l'entrée sur au moins un élément de commande comprend un mouvement de rotation et/ou un mouvement de pression sur un interrupteur tournant sensible à la pression.

10. Procédé suivant l'une des revendications 7 à 9, **caractérisé en ce qu'**il y a dans l'unité informatique au moins un paramètre d'état en cours de la table de patient et on crée dans l'unité informatique l'instruction de commande en fonction du au moins un paramètre d'état en cours de la table de patient.

11. Procédé suivant l'une des revendications 7 à 10, **caractérisé en ce que** l'instruction de commande créée comprend un sens d'un mouvement de la table de patient, dans lequel le sens du mouvement de la table de patient dépend d'un sens de rotation de l'élément de commande.

12. Produit de programme d'ordinateur, qui comprend un programme et qui peut être chargé directement dans une mémoire d'une unité informatique programmable d'une commande de table de patient d'une installation de résonance magnétique suivant la revendication 1, comprenant des moyens de programme pour exécuter un procédé de commande d'un mouvement d'une table de patient d'une installation de résonance magnétique suivant l'une des revendications 7 à 11, lorsque le programme est exécuté dans l'unité informatique.
